(19) Europäisches Patentamt
European Patent Office
Office européen des brevets

(11) Veröffentlichungsnummer: **0 126 226**
**A2**

# (12) EUROPÄISCHE PATENTANMELDUNG

(21) Anmeldenummer: **84102656.0**

(22) Anmeldetag: **12.03.84**

(51) Int. Cl.³: **C 07 D 231/18**, A 01 N 47/40

(30) Priorität: **24.03.83 DE 3310831**

(43) Veröffentlichungstag der Anmeldung: **28.11.84**
**Patentblatt 84/48**

(84) Benannte Vertragsstaaten: **AT BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Maurer, Fritz, Dr., Roeberstrasse 8,**
**D-5600 Wuppertal 1 (DE)**
Erfinder: **Homeyer, Bernhard, Dr., Obere Strasse 28,**
**D-5090 Leverkusen 3 (DE)**
Erfinder: **Becker, Benedikt, Dr.,**
**Metzkausenerstrasse 14, D-4020 Mettmann (DE)**

(54) **N-(alpha-Cyanalkylsulfenyl)-O-(pyrazol-4-yl)-carbamate, Verfahren zu ihrer Herstellung und ihre Verwendung als Schädlingsbekämpfungsmittel.**

(57) Die Erfindung betrifft neue N-(α-Cyanalkylsulfenyl)-O-(pyrazol-4-yl)-carbamate der allgemeinen Formel (I)

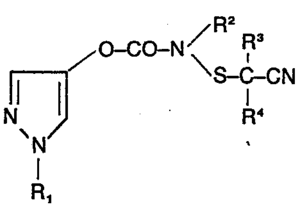

(I)

in welcher
$R^1$ für Alkyl oder Cycloalkyl,
$R^2$ für Alkyl und
$R^3$ und $R^4$ jeweils für Alkyl oder gemeinsam für einen zweifach gebundenen Alkylenrest stehen,
welche als Schädlingsbekämpfungsmittel verwendet werden können.

EP 0 126 226 A2

0126226

BAYER AKTIENGESELLSCHAFT
5090 Leverkusen, Bayerwerk
Konzernverwaltung RP
Patentabteilung

Rt/S/by-c
Typ Ib

BEZEICHNUNG GEÄNDERT
siehe Titelseite

Carbamate

Die Erfindung betrifft neue N-(α-Cyanalkylsulfenyl)-O-(pyrazol-4-yl)-carbamate, mehrere Verfahren zu ihrer Herstellung sowie ihre Verwendung in Schädlingsbekämpfungsmitteln, insbesondere in Insektiziden, Akariziden und Nematiziden.

Es ist bekannt, daß bestimmte N-Sulfenyl-O-pyrazolylcarbamate, wie beispielsweise das O-(1-Isopropyl-pyrazol-4-yl)-N-methyl-N-(4-methylphenylsulfenyl)-carbamat oder das O-(1-Isopropylpyrazol-4-yl)-N-dichlorfluormethyl-sulfenyl-N-methyl-carbamat oder das O-(1-t-Butylpyrazol-4-yl)-N-methyl-N-trichlormethyl-sulfenyl-carbamat oder das O-(1-t-Butylpyrazol-4-yl-N-methyl-N-phenylsulfenyl-carbamat oder das O-(t-Butylpyrazol-4-yl)-N-(t-butylsulfenyl)-N-methyl-carbamat insektizide Eigenschaften aufweisen (vgl. EP 43 917).

Die insektizide Wirkung dieser Verbindungen ist jedoch insbesondere bei niedrigen Wirkstoffkonzentrationen und -aufwandmengen nicht immer zufriedenstellend.

Es wurden neue N-(α-Cyanalkylsulfenyl)-O-(pyrazol-4-yl)-carbamate der allgemeinen Formel (I)

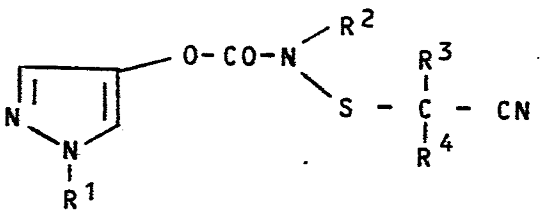

(I)

in welcher

$R^1$ für Alkyl oder Cycloalkyl,

$R^2$ für Alkyl und

$R^3$ und $R^4$ jeweils für Alkyl oder gemeinsam für einen zweifach gebundenen Alkylenrest stehen,

gefunden.

Weiterhin wurde gefunden, daß man die neuen N-(α-Cyanalkylsulfenyl)-O-(pyrazol-4-yl)-carbamate der allgemeinen Formel (I)

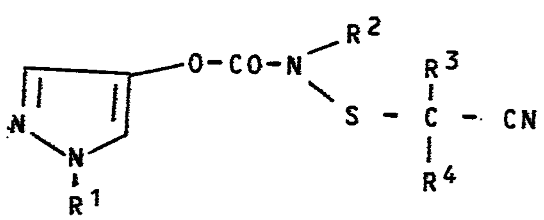

(I)

in welcher

$R^1$ für Alkyl oder Cycloalkyl,

$R^2$ für Alkyl und

$R^3$ und $R^4$ jeweils für Alkyl oder gemeinsam für einen zweifach gebundenen Alkylenrest stehen,

erhält, wenn man

a) 4-Hydroxypyrazole der Formel (II),

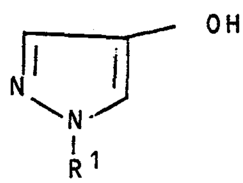

(II)

in welcher

$R^1$ die oben angegebene Bedeutung hat,

mit N-sulfenylierten Carbamoylhalogeniden der Formel

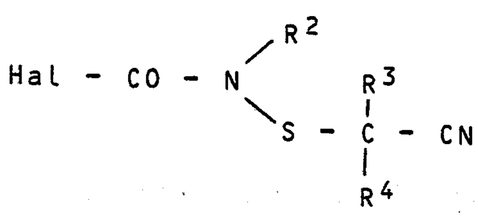

(III)

in welcher

$R^2$, $R^3$ und $R^4$ die oben angegebene Bedeutung haben und

Hal für Halogen steht,

gegebenenfalls unter Verwendung eines Verdünnungsmittels und gegebenenfalls in Gegenwart eines Säureakzeptors umsetzt, oder wenn man

b) O-Pyrazol-4-ylcarbamate der Formel (IV)

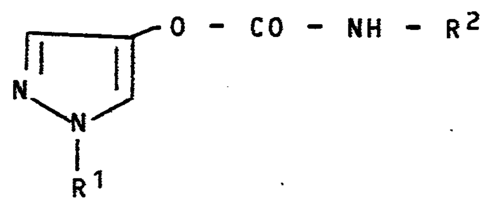

(IV)

in welcher

$R^1$ und $R^2$ die oben angegebene Bedeutung haben,

mit Sulfenylhalogeniden der Formel (V)

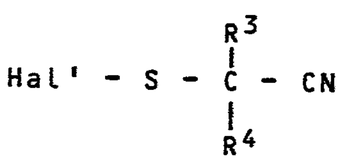

(V)

in welcher

$R^3$ und $R^4$ die oben angegebene Bedeutung haben und

Hal' für Halogen steht,

gegebenenfalls unter Verwendung eines Verdünnungsmittels und gegebenenfalls in Gegenwart eines Säureakzeptors umsetzt.

Die neuen N-(α-Cyanalkylsulfenyl)-O-(pyrazol-4-yl)-carbamate der Formel (I) zeichnen sich durch hohe Wirksamkeit als Schädlingsbekämpfungsmittel, insbesondere durch hohe insektizide und nematizide Wirksamkeit aus.

Überraschenderweise zeigen die erfindungsgemäßen N-(α-Cyanalkylsulfenyl)-O-(pyrazol-4-yl)-carbamate der Formel (I) eine erheblich höhere insektizide und nematizide Wirksamkeit als bekannte Verbindungen ähnlicher Konstitution und gleicher Wirkungsrichtung, wie z.B. die bereits erwähnten Verbindungen O-(1-Isopropylpyrazol-4-yl)-N-methyl-N-(4-methylphenylsulfenyl)-carbamat, O-(1-Isopropylpyrazol-4-yl)-N-dichlorfluormethyl-sulfenyl-N-methyl-carbamat, O-(1-t-Butyl-pyrazol-4-yl)-N-methyl-N-trichlormethyl-sulfenyl-carbamat, O-(t-Butylpyrazol-4-yl)-N-methyl-N-phenylsulfenyl-carbamat und O-(1-t-Butylpyrazol-4-yl)-N-(t-butylsulfenyl)-N-methyl-carbamat.

Alkyl $R^1$, $R^2$ und $R^3$ bedeutet geradkettiges oder verzweigtes Alkyl mit vorzugsweise 1 bis 8, insbesondere 1 bis 6 und besonders bevorzugt 1 bis 4 Kohlenstoffatomen, wobei beispielhaft Methyl, Ethyl, n- und i-Propyl, n-, i-, s- und t-Butyl und 1,1-Dimethylpropyl genannt.

Cycloalkyl $R^1$ bedeutet vorzugsweise Cycloalkyl mit 3 bis 7, insbesondere 3, 5 oder 6 Kohlenstoffatomen, wobei beispielhaft Cyclopropyl, Cyclobutyl, Cyclopentyl, Cyclohexyl und Cycloheptyl genannt seien.

Der durch $R^3$ und $R^4$ gebildete Alkylenrest kann geradkettig oder verzweigt sein und enthält vorzugsweise 4 bis 7 Kohlenstoffatome, wobei beispielsweise Ethylen, Propylen und Butylen genannt seien.

Hal und Hal' stehen für Fluor, Chlor, Brom oder Jod, vorzugsweise für Fluor, Chlor oder Brom, insbesondere für Fluor oder Chlor.

Die erfindungsgemäßen N-($\alpha$-Cyanalkylsulfenyl)-O-(pyrazol-4-yl)-carbamate sind durch die Formel (I) allgemein definiert.

Bevorzugt sind Verbindungen der Formel (I), bei denen

$R^1$ für geradkettiges oder verzweigtes Alkyl mit bis zu 8 Kohlenstoffatomen oder für Cycloalkyl mit 3 bis 8 Kohlenstoffatomen steht,

$R^2$ für geradkettiges oder verzweigtes Alkyl mit bis zu 6 Kohlenstoffatomen steht und

$R^3$ und $R^4$ jeweils für geradkettiges oder verzweigtes Alkyl mit bis zu 6 Kohlenstoffatomen stehen oder gemeinsam für einen zweifach verknüpften Alkylenrest mit 4 bis 7 Kohlenstoffatomen stehen.

Besonders bevorzugt sind Verbindungen der Formel (I), bei denen

$R^1$ für geradkettiges oder verzweigtes Alkyl mit 1 bis 6 Kohlenstoffatomen oder für Cyclopropyl, Cyclopentyl, Cyclohexyl oder Cycloheptyl steht,

$R^2$ für geradkettiges oder verzweigtes Alkyl mit 1 bis 4 Kohlenstoffatomen steht und

$R^3$ und $R^4$ jeweils für geradkettiges oder verzweigtes Alkyl mit 1 bis 4 Kohlenstoffatomen stehen oder gemeinsam für einen Rest $-(CH_2)_n-$ stehen, wobei n für die Zahlen 4 oder 5 steht.

Verwendet man als Ausgangsstoffe beispielsweise 4-Hydroxy-1-isopropyl-pyrazol und N-(2-Cyanprop-2-yl-sulfenyl)-N-methyl-carbamoylfluorid, so kann der Reaktionsablauf des erfindungsgemäßen Verfahrens a) durch das folgende Formelschema dargestellt werden:

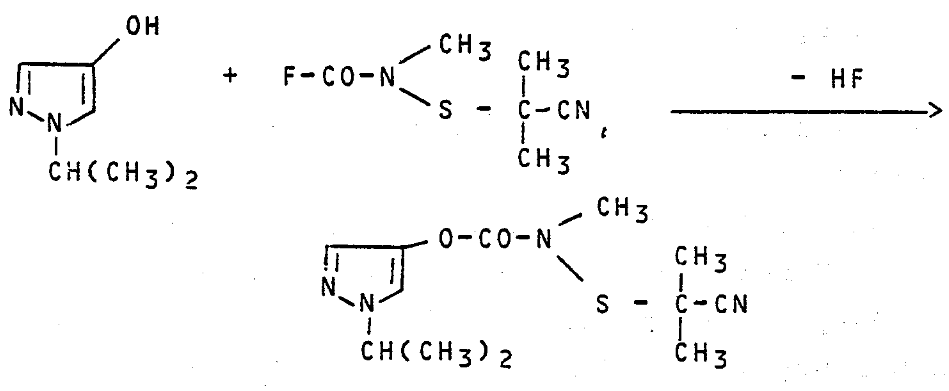

Verwendet man als Ausgangsstoffe beispielsweise O-(1-t-Butylpyrazol-4-yl)-N-methyl-carbamat und 2-Cyan-prop-2-yl-sulfenylchlorid, so kann der Reaktionsablauf des erfindungsgemäßen Verfahrens b) durch das folgende Formelschema dargestellt werden:

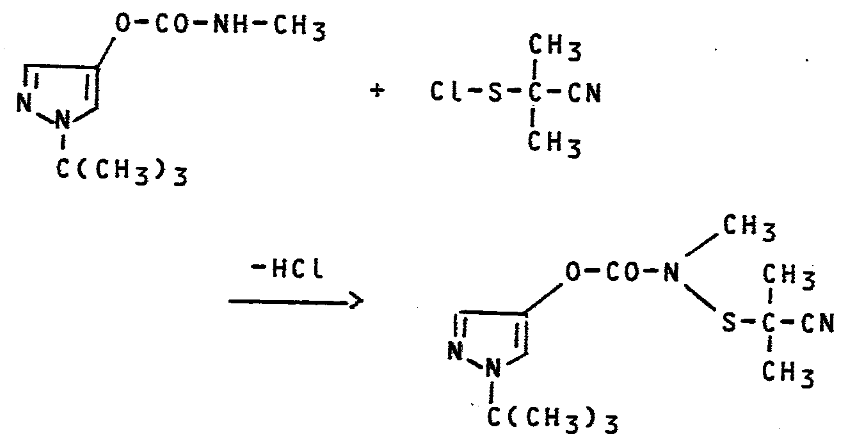


Die für das erfindungsgemäße Verfahren als Ausgangsstoffe zu verwendenden 4-Hydroxypyrazole sind durch die Formel (II) allgemein definiert. In dieser Formel (II) besitzt $R^1$ die gleiche Bedeutung, wie sie bei der Beschreibung der erfindungsgemäßen Stoffe der Formel (I) angegeben wurde.

Als Beispiele für Verbindungen der Formel (II) seien genannt:
1-Methyl-, 1-Ethyl-, 1-n-Propyl-, 1-iso-Propyl-, 1-(1,1-Dimethyl-propyl)-, 1-(2,2-Dimethylpropyl)-, 1-n-Butyl-, 1-sec.-Butyl-, 1-tert.-Butyl-, 1-(1-Methylbutyl)-, 1-(2-Methylbutyl)-, 1-(3-Methylbutyl)-, 1-n-Pentyl-, 2-(1-Ethylpropyl)-, 1-Cyclopropyl-, 1-Cyclobutyl-, 1-Cyclopentyl-, 1-Cyclohexyl-4-hydroxy-pyrazole.

Die 4-Hydroxypyrazole der Formel (II) sind bekannt (vergleiche Liebigs Ann. Chem. 313, (1900), 17 und DE-OS 2 931 033).

Man erhält die 4-Hydroxy-pyrazole der Formel (II) beispielsweise durch Umsetzung der entsprechenden 4-Methoxypyrazole mit Bromwasserstoffsäure. Die Herstellung der 4-Methoxypyrazole kann auf bekannte Weise, beispielsweise durch Umsetzung von entsprechenden Hydrazinen mit 2-Methoxy-4-dimethyl-amino-acrolein erfolgen (vergleiche Archiv der Pharmazie 300, (1967), 704-708).

Die für das erfindungsgemäße Verfahren a) weiterhin als Ausgangsstoffe zu verwendenden N-sulfenylierten Carbamoylhalogenide sind durch die Formel (III) allgemein definiert. In dieser Formel (III) besitzen $R^2$, $R^3$ und $R^4$ die gleiche Bedeutung, wie sie bei der Beschreibung der erfindungsgemäßen Stoffe der Formel (I) angegeben wurde. Hal steht vorzugsweise für Fluor oder Chlor.

Als Beispiele für die Verbindungen der Formel (III) seien genannt:

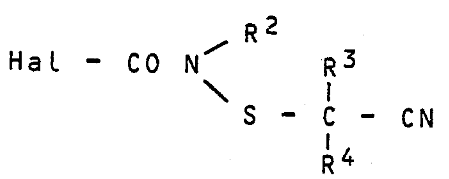

(III)

Hal = Fluor oder Chlor

| $R^2$ | $R^3$ | $R^4$ |
|---|---|---|
| $CH_3$ | $CH_3$ | $CH_3$ |
| $C_2H_5$ | $CH_3$ | $CH_3$ |
| $i-C_3H_7$ | $CH_3$ | $CH_3$ |
| $t-C_4H_9$ | $CH_3$ | $CH_3$ |
| $CH_3$ | $CH_3$ | $C_2H_5$ |
| $C_2H_5$ | $CH_3$ | $C_2H_5$ |
| $i-C_3H_7$ | $CH_3$ | $C_2H_5$ |
| $t-C_4H_9$ | $CH_3$ | $C_2H_5$ |
| $CH_3$ | $CH_3$ | $i-C_3H_7$ |
| $C_2H_5$ | $CH_3$ | $i-C_3H_7$ |
| $i-C_3H_7$ | $CH_3$ | $i-C_3H_7$ |
| $t-C_4H_9$ | $CH_3$ | $i-C_3H_7$ |
| $CH_3$ | $-CH_2-CH_2-CH_2-CH_2-$ | |
| $C_2H_5$ | $-CH_2-CH_2-CH_2-CH_2-$ | |
| $i-C_3H_7$ | $-CH_2-CH_2-CH_2-CH_2-$ | |
| $t-C_4H_9$ | $-CH_2-CH_2-CH_2-CH_2-$ | |
| $CH_3$ | $-CH_2-CH_2-CH_2-CH_2-CH_2-$ | |
| $C_2H_5$ | $-CH_2-CH_2-CH_2-CH_2-CH_2-$ | |

Die N-sulfenylierten Carbamoylhalogenide der Formel (III) sind bekannt (vgl. DE-OS 3 109 028 und EP 25 014).

Man erhält die sulfenylierten Carbamoylhalogenide der Formel (III) beispielsweise durch Umsetzung der entsprechenden Sulfenylhalogenide mit N-substituierten Carbamoylhalogeniden in Gegenwart eines Verdünnungsmittels, wie z.B. Toluol, und in Gegenwart eines Säureakzeptors, wie z.B. Triethylamin, bei Temperaturen zwischen -10 und 100°C.

Die für das erfindungsgemäße Verfahren b) als Ausgangsstoffe zu verwendenden O-Pyrazol-4-ylcarbamate sind durch die Formel (IV) allgemein definiert. In dieser Formel (IV) besitzen $R^1$ und $R^2$ die gleiche Bedeutung wie sie bei der Beschreibung der erfindungsgemäßen Stoffe der Formel (I) angegeben wurde.

Als Beispiele für die Verbindungen der Formel (IV) seien genannt:
N-n-Propyl-O-(1-isopropyl-pyrazol-4-yl)-, N-Ethyl-O-(1-tert.-butyl-pyrazol-4-yl)-, N-iso-Propyl-O-(1-isopropyl-pyrazol-4-yl)-, N-Ethyl-O-(1-isopropyl-pyrazol-4-yl)-, N-n-Propyl-O-(1-sec.-butyl-pyrazol-4-yl)-, N-Ethyl-O-(1-(2-methyl-2-butyl)-pyrazol-4-yl)-, N-n-Propyl-O-(1-(2-methyl-2-butyl)-pyrazol-4-yl)-, N-Ethyl-O-(1-n-propyl-pyrazol-4-yl)-, N-Isopropyl-O-(1-(2-methyl-2-butyl)-pyrazol-4-yl)-, N-Ethyl-O-(1-ethyl-pyrazol-4-yl)-, N-Ethyl-O-(1-cyclohexyl-pyrazol-4-yl)-, N-Ethyl-O-(1-sec.-butyl-pyrazol-4-yl)-, N-Ethyl-O-(1-methyl-pyrazol-4-yl)-, N-Ethyl-O-(1-cyclopropyl-pyrazol-4-yl)- und N-n-Propyl-(1-tert.-butyl-pyrazol-4-yl)-carbamat.

Die Verbindungen der Formel (IV) sind bekannt (vergl. DE-OS 3114 833; EP-OS 43 917).

Man erhält die Verbindungen der Formel (IV) z.B. durch Umsetzung von 4-Hydroxy-pyrazolen der Formel (II) mit entsprechenden Isocyanaten in Gegenwart eines Verdünnungsmittels, wie z.B. Aceton, Methylenchlorid oder Toluol und gegebenenfalls in Gegenwart eines Katalysators wie z.B. Triethylamin bei Temperaturen zwischen 10 und 80°C, oder mit Phosgen und den entsprechenden Aminen in Gegenwart eines Verdünnungsmittels wie z.B. Toluol und gegebenenfalls in Gegenwart eines Säureakzeptors wie z.B. Triethylamin bei Temperaturen zwischen -10 und 80°C.

Die für das erfindungsgemäße Verfahren b) weiterhin als Ausgangsstoffe zu verwendenden Sulfenylhalogenide sind durch die Formel (V) allgemein definiert. In dieser Formel (V) haben $R^3$ und $R^4$ die gleiche Bedeutung, wie sie bei der Beschreibung der erfindungsgemäßen Stoffe der Formel (I) angegeben wurde. Hal' steht vorzugsweise für Fluor oder Chlor.

Als Beispiele für Verbindungen der Formel (V) seien genannt:

2-Cyanprop-2-yl-, 2-Cyanbut-2-yl-, 2-Cyanpent-2-yl-, 2-Cyanhex-2-yl-, 3-Cyanpent-3-yl-, 3-Cyanhex-3-yl-, 3-Cyanhept-3-yl-, 4-Cyanhept-3-yl-, 2-Cyan-3-methylbut-2-yl-, 2-Cyan-3,3-dimethylbut-2-yl-, 3-Cyan-2,4-dimethylpent-3-yl-, 1-Cyan-cyclopentyl-, 1-Cyancyclohexyl-sulfenylfluorid bzw. -sulfenylchlorid.

Die Sulfenylhalogenide der Formel (V) sind bekannt (vgl. US-PS 3 832 378).

Die erfindungsgemäßen Herstellungsverfahren a) und b) werden vorzugsweise unter Verwendung von Verdünnungsmitteln durchgeführt. Als Verdünnungsmittel kommen praktisch alle inerten organischen Lösungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone, wie Aceton, Methylethyl-, Methylisopropyl- und Methyl-isobutyl-keton-, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid. Vorzugsweise wird Toluol als Verdünnungsmittel verwendet.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin und Diazabicycloundecen. Triethylamin wird bevorzugt als Säureakzeptor eingesetzt. Die Reaktionstemperatur kann innerhalb eines größeren Bereiches variiert werden. Im allgemeinen arbeitet man zwischen -20 und 100°C; vorzugsweise bei 0 bis 80°C.

Die erfindungsgemäßen Verfahren a) und b) werden im allgemeinen bei Normaldruck durchgeführt.

Zur Durchführung der erfindungsgemäßen Verfahren a) und b) werden die Ausgangsstoffe gewöhnlich in äquivalenten Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man gegebenenfalls ein mit Wasser nicht mischbares organisches Lösungsmittel, z.B. Toluol, zu und arbeitet die organische Phase wie üblich durch Waschen, Trocknen und Abdestillieren des Lösungsmittels auf. Man erhält so die Produkte in öliger oder kristalliner Form. Zur Charakterisierung dient der Schmelzpunkt oder der Brechungsindex.

Die neuen Verbindungen fallen zum Teil in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen N-($\alpha$-Cyanalkylsulfenyl)-O-(pyrazol-4-yl)-carbamate der Formel (I) zeigen neben einer guten insektiziden und nematiziden Wirkung auch eine gute Wirkung gegen Hygiene- und Vorratsschädlinge.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.

Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe,

Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Herstellungsbeispiele

### Beispiel 1

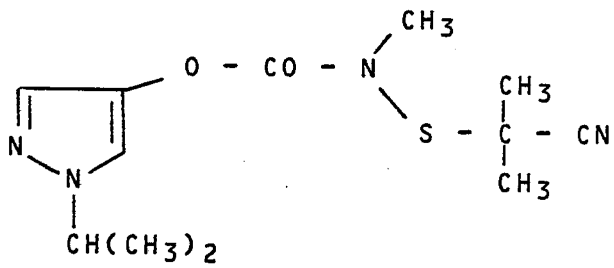

Zu einer Mischung aus 9,45 g (0,075 Mol) 4-Hydroxy-1-isopropyl-pyrazol, 8,5 g (0,085 Mol) Triethylamin und 100 ml Toluol tropft man bei 25°C 12 g (0,075 Mol) N-(2-Cyanprop-2-ylsulfenyl)-N-methyl-carbamoylfluorid. Nach beendeter Zugabe rührt man 18 Stunden bei Raumtemperatur. Zur Aufarbeitung wird die Reaktionsmischung 2 mal mit Wasser gewaschen, über Natriumsulfat getrocknet und im Vakuum vom Lösungsmittel befreit. Man erhält 18 g (85 % der Theorie) an N-(2-Cyanprop-2-ylsulfenyl)-O-(1-isopropylpyrazol-4-yl)-N-methyl-carbamat in Form eines gelben Öls mit dem Brechungsindex $n_D^{21}$ = 1,5070.

In entsprechender Weise und gemäß den allgemeinen Herstellungsbedingungen erhält man die folgenden Verbindungen der Formel (I)

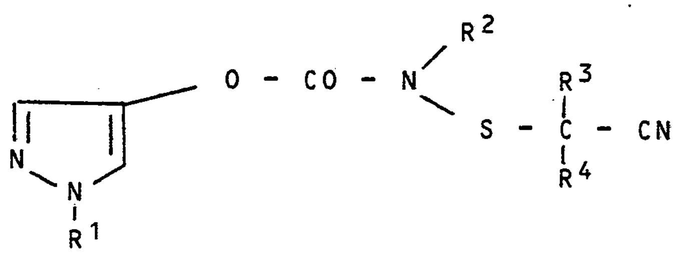

(I)

| Bsp. Nr. | $R^1$ | $R^2$ | $R^3$ | $R^4$ | Brechungs-index |
|---|---|---|---|---|---|
| 2 | $t-C_4H_9$ | $CH_3$ | $CH_3$ | $CH_3$ | $n_D^{21}$ 1,5050 |
| 3 | $CH_3$ | $CH_3$ | $CH_3$ | $CH_3$ | |
| 4 | $C_2H_5$ | $CH_3$ | $CH_3$ | $CH_3$ | |
| 5 | $t-C_4H_9$ | $C_2H_5$ | $CH_3$ | $CH_3$ | |
| 6 | $C_2H_5-\overset{CH_3}{\underset{CH_3}{C}}-$ | $CH_3$ | $CH_3$ | $CH_3$ | |
| 7 | $c-C_6H_{11}$ | $CH_3$ | $CH_3$ | $CH_3$ | |
| 8 | $t-C_4H_9$ | $CH_3$ | $-CH_2CH_2-CH_2-CH_2-$ | | |
| 9 | $t-C_4H_9$ | $i-C_3H_7$ | $CH_3$ | $CH_3$ | |
| 10 | $s-C_4H_9$ | $CH_3$ | $CH_3$ | $CH_3$ | |

Anwendungsbeispiele

In den nachstehenden Anwendungsbeispielen werden die folgenden Verbindungen als Vergleichssubstanzen eingesetzt:

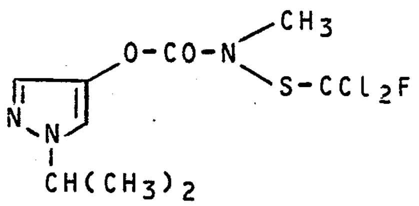

(A)

N-Dichlorfluormethylsulfenyl-O-(1-isopropylpyrazol-4-yl)-N-methyl-carbamat

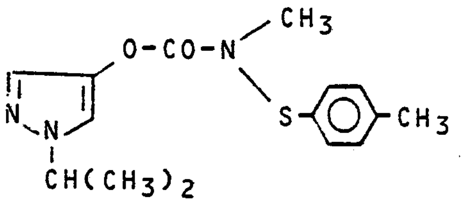

(B)

O-(1-Isopropylpyrazol-4-yl)-N-methyl-N-(4-methylphenylsulfenyl)-carbamat

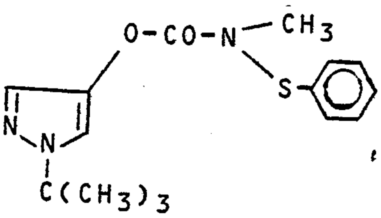

(C)

O-(1-t-Butylpyrazol-4-yl)-N-methyl-N-phenylsulfenyl-carbamat

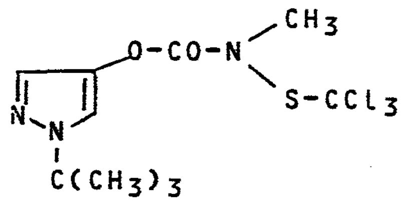

(D)

O-(1-t-Butylpyrazol-4-yl)-N-methyl-N-trichlormethyl-sulfenyl-carbamat

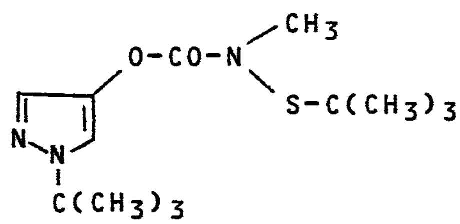

(E)

O-(1-t-Butylpyrazol-4-yl)-N-(t-butylsulfenyl)-N-methylcarbamat.

Beispiel A

Grenzkonzentrations-Test / Bodeninsekten

Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigt z.B. die Verbindung gemäß Herstellungsbeispiel (2) überlegene Wirkung gegenüber dem Stand der Technik.

0126226

Beispiel B

Grenzkonzentrations-Test / Wurzelsystemische Wirkung

Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2.

Beispiel C

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2.

Beispiel D

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigt z.B. die Verbindung gemäß Herstellungsbeispiel (2) überlegene Wirkung gegenüber dem Stand der Technik.

Beispiel E

Drosophila-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese naß auf die Öffnung eines Glasgefäßes, in dem sich 50 Taufliegen (Drosophila melanogaster) befinden und bedeckt es mit einer Glasplatte.

Nach der gewünschten Zeit bestimmt man die Abtötung in %. Dabei bedeutet 100 %, daß alle Fliegen abgetötet wurden; 0 % bedeutet, daß keine Fliegen abgetötet wurden.

Bei diesem Test zeigen z.B. die folgenden Verbindungen der Herstellungsbeispiele überlegene Wirksamkeit gegenüber dem Stand der Technik: 2.

Beispiel F

Doralis-Test (systemische Wirkung)

Lösungsmittel: 3 Gewichtsteile
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia faba), die stark von der schwarzen Bohnenlaus (Doralis fabae) befallen sind, angegossen, so daß die Wirkstoffzubereitung in den Boden eindringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1 und 2.

Beispiel G

Tetranychus-Test (resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2.

Patentansprüche

1. N-(α-Cyanalkylsulfenyl)-O-(pyrazol-4-yl)carbamate der allgemeinen Formel (I)

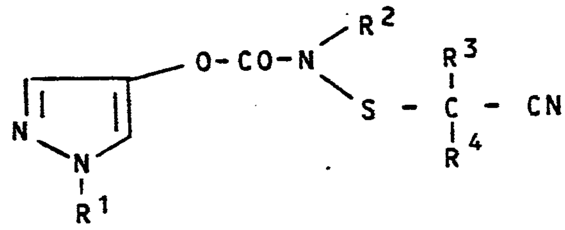

(I)

in welcher

$R^1$ für Alkyl oder Cycloalkyl,

$R^2$ für Alkyl und

$R^3$ und $R^4$ jeweils für Alkyl oder gemeinsam für einen zweifach gebundenen Alkylrest stehen.

2. Verbindungen gemäß Anspruch 1, in welchen

$R^1$ für geradkettiges oder verzweigtes Alkyl mit bis zu 8 Kohlenstoffatomen oder für Cycloalkyl mit 3 bis 8 Kohlenstoffatomen steht,

$R^2$ für geradkettiges oder verzweigtes Alkyl mit bis zu 6 Kohlenstoffatomen steht und

$R^3$ und $R^4$ jeweils für geradkettiges oder verzweigtes Alkyl mit bis zu 6 Kohlenstoffatomen stehen oder gemeinsam für einen zweifach verknüpften Alkylenrest mit 4 bis 7 Kohlenstoffatomen stehen.

3. Verbindungen gemäß Anspruch 1, in welchen

$R^1$ für geradkettiges oder verzweigtes Alkyl mit 1 bis 6 Kohlenstoffatomen oder für Cyclopropyl, Cyclopentyl, Cyclohexyl oder Cycloheptyl steht,

$R^2$ für geradkettiges oder verzweigtes Alkyl mit 1 bis 4 Kohlenstoffatomen steht und

$R^3$ und $R^4$ jeweils für geradkettiges oder verzweigtes Alkyl mit 1 bis 4 Kohlenstoffatomen stehen oder gemeinsam für einen Rest $-(CH_2)_n-$ stehen, wobei n für die Zahlen 4 oder 5 steht.

4. Verbindung der Formel

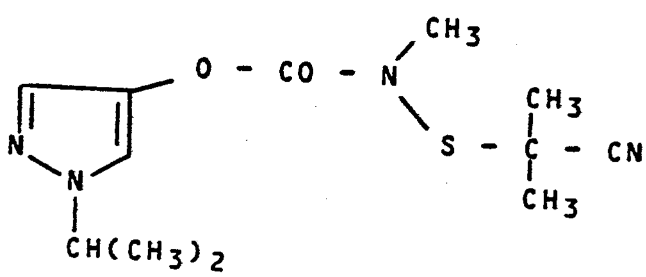

5. Verbindung der Formel

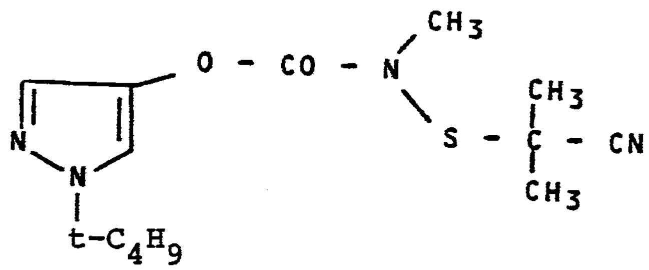

6. Verfahren zur Herstellung von N-(α-Cyanalkylsulfenyl)-O-(pyrazol-4-yl)-carbamaten der allgemeinen Formel (I)

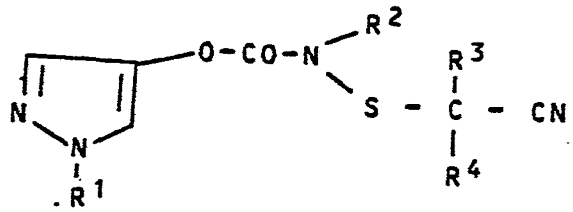

(I)

in welcher

$R^1$ für Alkyl oder Cycloalkyl,

$R^2$ für Alkyl und

$R^3$ und $R^4$ jeweils für Alkyl oder gemeinsam für einen zweifach gebundenen Alkylenrest stehen,

dadurch gekennzeichnet, daß man

a) 4-Hydroxypyrazole der Formel (II)

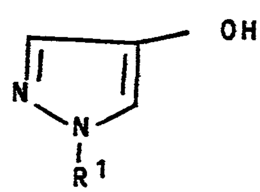

(II)

in welcher

$R^1$ die oben angegebene Bedeutung hat,

mit N-sulfenylierten Carbamoylhalogeniden der Formel

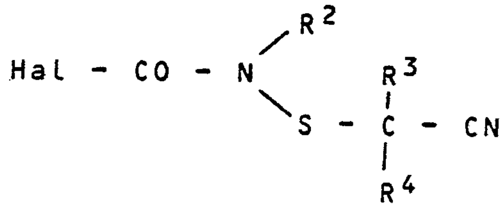

(III)

in welcher

$R^2$, $R^3$ und $R^4$ die oben angegebene Bedeutung haben und

Hal für Halogen steht,

gegebenenfalls unter Verwendung eines Verdünnungsmittels und gegebenenfalls in Gegenwart eines Säureakzeptors umsetzt, oder

b) O-Pyrazol-4-ylcarbamate der Formel (IV)

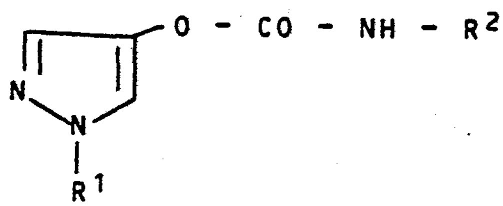

(IV)

in welcher

$R^1$ und $R^2$ die oben angegebene Bedeutung haben,

mit Sulfenylhalogeniden der Formel (V)

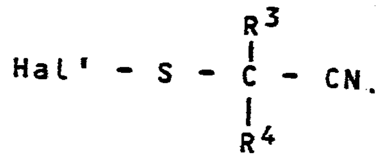

(V)

in welcher

$R^3$ und $R^4$ die oben angegebene Bedeutung haben und

Hal' für Halogen steht,

gegebenenfalls unter Verwendung eines Verdünnungsmittels und gegebenenfalls in Gegenwart eines Säureakzeptors umsetzt.

7. Schädlingsbekämpfungsmittel, gekennzeichnet durch einen Gehalt an mindestens einer Verbindung der Formel (I) gemäß den Ansprüchen 1 oder 6.

8. Verwendung von Verbindungen der Formel (I) gemäß den Ansprüchen 1 oder 6 zur Bekämpfung von Schädlingen, insbesondere Insekten, Spinnentieren und Nemathoden.

9. Verfahren zur Bekämpfung von Schädlingen, dadurch gekennzeichnet, daß man Verbindungen der Formel (I)

gemäß den Ansprüchen 1 oder 6 auf die Schädlinge, vorzugsweise Insekten, Spinnentiere oder Nematoden oder ihren Lebensraum einwirken läßt.

10. Verfahren zur Herstellung von Schädlingsbekämpfungsmitteln, dadurch gekennzeichnet, daß man Verbindungen der Formel (I) gemäß den Ansprüchen 1 oder 6 mit Streckmitteln und/oder oberflächenaktiven Mitteln vermischt.